Europäisches Patentamt

European Patent Office

Office européen des brevets

(19)

(11) Publication number: **0 081 131**
**B1**

(12) **EUROPEAN PATENT SPECIFICATION**

(45) Date of publication of patent specification: **09.04.86**

(51) Int. Cl.⁴: **A 61 F 5/46**

(21) Application number: **82110756.2**

(22) Date of filing: **21.11.82**

(54) **Contraceptive vaginal barrier.**

(30) Priority: **09.12.81 IT 2548681**

(43) Date of publication of application:
**15.06.83 Bulletin 83/24**

(45) Publication of the grant of the patent:
**09.04.86 Bulletin 86/15**

(84) Designated Contracting States:
**AT BE CH DE FR GB LI LU NL SE**

(56) References cited:
**CH-A- 372 793**
**FR-A- 440 230**
**US-A-2 823 669**
**US-A-3 128 767**
**US-A-4 031 886**
**US-A-4 108 180**

(73) Proprietor: **Boschetti, Enrica**
**171, Via Melchiorre Gioia**
**I-20125 Milano (IT)**

(72) Inventor: **Boschetti, Enrica**
**171, Via Melchiorre Gioia**
**I-20125 Milano (IT)**

(74) Representative: **Ferraiolo, Ruggero**
**Via Napo Torriani, 10**
**I-20124 Milano (IT)**

Courier Press, Leamington Spa, England.

## Description

The present invention relates to a contraceptive vaginal barrier.

Non-chemical contraceptive means are known, referred to as diaphragms, that are generally associated, immediately before using, with spermicide and/or medicinal substances. Generally, these diaphragms comprise an outer structure that acts as a frame of a rubber cap peripherally fixed to the outer structure. These diaphragms are of circular or oval shape and the outer structure is generally contained in a plane. Moreover, the outer structure may be peripherically deformed by a user or by a therapist in order to anantomically adapt it.

The following patents disclosing vaginal diaphragms are known to the applicant: U.S. patents No 961,880 — 2,087,610 — 2,443,943 — 2,463,356 — 2,529,363 — 2,638,896 — 2,664,082 — 2,676,589 — 2,823,669 — 2,875,755 — 3,060,931 — 3,128,762 — 3,169,894 — 4,031,886 — 4,108,180 — and the German patent No 557,914. Only some of the above patents are cited herebelow as being deemed to be more pertinent to the present invention.

Among the diaphragms having a circular outer structure contained in a plane it is cited the one disclosed in the U.S. patent No 2,443,943 of T. Young wherein the outer structure comprises two separate steel segments housed into a spiral spring incorporated into a rubber sleeve. Also it is cited the diaphragm disclosed in the U.S. patent No 2,463,356 of J. T. Clark wherein the oval outer structure is a rubber sleeve wherein two flat springs are located, each having its ends superposed with corresponding ends of the other spring in order to provide for an adjustable diaphragm. Also it is cited the diaphragm disclosed in the U.S. patent No 2,823,669 of E. Kunnas, of circular shape, comprising an outer structure wherein two separate steel segments are connected each other by two small spiral springs wherein their pointed ends are engaged, all these elements being housed within a larger closed spiral spring, in turn held into a rubber sleeve, so as to provide for a diaphragm that may be folded for introduction in the anatomic cavity. The Italian model and patent applications in the name of Enrica Boschetti, No 22,238 B/78, No 25, 294 A/78 and No 19,918 A/79 are also cited, respectively concerning: a circular or oval diaphragm wherein the cap is connected with the outer structure in correspondence with the middle plane of the outer structure; an oval diaphragm wherein the inner armour of the outer structure may be extended and shortened in order to modify the dimensions of the diaphragm; a diaphragm whose outer structure comprises a spiral spring incorporating flexible metal bars and is of oval or elliptical cross section having the main axis substantially parallel with an axis perpendicular with the diaphragm plane.

Finally, a pessary disclosed in the U.S. patent No 4,031,886 of V. B. Morhenn is cited that is formed by an outer structure having in the side view a specific shape for receiving the human cervix and a bag adapted to receive that outer structure. The bag has two generally planar walls each having four edges and may be spread with medicaments.

The main drawbacks to the pessary disclosed in U.S. patent No 4,031,886 are that the outer structure must be orientated in a certain direction when it is introduced into the bag, that the shape of the diaphragm does not allow adjustments in the shape and dimensions and that the pessary must be applied into the anatomic cavity in a certain direction.

The contraceptive vaginal barrier according to the present invention comprises at least three elements: an outer structure, of circular or oval shape, whose inscribed surface is contained in a plane; an expandable container preferably made of natural or synthetic woven material, but alternatively of flexible plastic, rubber or sponge wherein a corresponding outer structure may be introduced and closed; a spermicide and/or medicinal substance applied to the inner and outer surfaces of the container.

Herebelow, an outer structure will be referred to as ring and a spermicide and/or medicinal substance will be referred to as medicament.

The ring is completely identical or similar to a ring of any known circular or oval diaphragm.

Preferably, the ring is composed of an outer sleeve made of natural rubber or equivalent synthetic material wherein means are incorporated for allowing modification of the peripheral dimensions of the outer sleeve and/or imparting a desired shape to the outer sleeve.

Alternatively, the ring is simply a body of annular shape having circular or oval cross section, made of an elastic material such as, rubber, plastic or steel.

The container is a bag having substantially the shape of an ellipse, if considered flat, and of an ellipsoid if considered three-dimensional, the bag being provided with a first aperture for introducing a ring and with means for closing said aperture, the bag being adapted to receive a ring that may be located into the container with its plane containing the main or the minor axis of the container, the container and the ring being geometrically associable so that the ring introduced in the container stretches the periphery thereof along the contact path in order to form a cup-shaped element having a periphery substantially coincident with the periphery of the ring and with the two container walls superposed, kept close each to another also due to the adherence between the layers of the medicament applied to the opposite surfaces.

The container may be provided with a second aperture, closed by a means that may be removed easily and rapidly for drawing out the ring contained therein.

The advantages of the diaphragm according to this present invention consist of the fact that it may be prepared for use easily and that it may be deformed to adjust the shape for the anatomic requirements.

An embodiment of the invention is illustrated, as an example only, with reference to the enclosed drawings wherein:

FIG. 1 shows an oval ring having circular cross section,

FIG. 2 shows a container adapted to receive a ring as illustrated in FIG. 1,

FIG. 3 shows a vaginal contraceptive barrier obtained as an association of said ring and container,

FIG. 4 shows an alternative ring of oval shape.

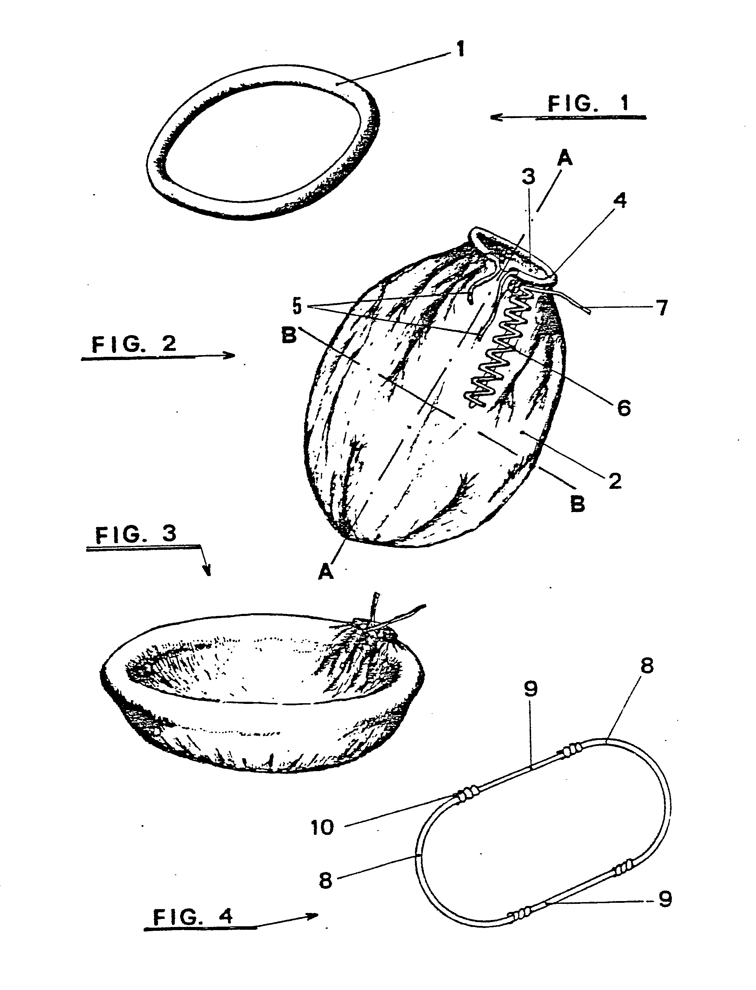

The ring as shown in Figure 1 has the features of the outer structure of the diaphragm disclosed in the U.S patent No 2,823,669 of Kunnas wherein two separate segments made of steel rod are connected each with another by means of two small spiral rings, this assembly being incorporated in a larger closed spiral spring, in turn incorporated in a rubber sleeve. The container 2 is a bag made of cotton fabric, very soft and absorbent, that comprises an aperture 3 into which the ring 1 may be introduced, a tubular housing 4 all around the aperture 3 into which housing a yarn 5 is passed whose ends extend outside in order to be pulled and knotted for permanently closing the aperture 3, a yarn 6 closing the edges of an aperture made along some centimeters on a container wall, the yarn being provided with an end 7 that, when pulled, makes the aperture open in order to allow a easy and rapid removing of the ring 1 from the container 2.

It will be realized that the ring may be alternatively formed by a closed spiral spring or by a steel solid ring, or by a rubber ring formed around a core of steel or of another material of similar mechanical properties; the ring may be also formed by two small arcs of steel or of material having similar mechanical properties, the small arcs being diametrically opposed and having each couple of opposite ends connected each other by means of an articulated joint through an elastic intermediate structure. The latter alternative is illustrated in the Figure 4 wherein the two small steel arcs 8 are connected each other, through the elastic steel bars 9, by means of four articulated joints made by engaging corresponding ends of a small arc 8 and of a bar 9 into a spiral spring 10.

Also it will be realized that the ring, which is always an elastic structure, is conveniently folded by the fingers for introduction into the container through the aperture. Once the ring is in the container, it takes again its natural shape or the shape previously imparted from a user or a therapist by conveniently deforming the inner means provided for on purpose and the ring stretches the container walls to form a vaginal barrier similar with a known diaphragm, as illustrate in the Figure 3 which also shows the two container walls cup-shaped, one on the other. According to Figure 3, the ring is located in the container with its plane containing the main axis A—A of the container shown in Figure 2, but alternatively the ring may be located in the container with its plane containing the minor axis B—B of the container shown in Figure 2.

## Claims

1. A contraceptive vaginal barrier comprising a circular or oval ring (1); a disposable container (2) in the form of a bag made of a flexible material having an aperture (3) through which the ring may be introduced and means (4, 5) for closing the aperture, the ring and the container being such that the ring stretches the container when introduced therein so as to form a substantially cup-shaped element having a periphery corresponding to the periphery of the ring and with the two container walls superimposed on one another; and a spermicidal and/or medicinal substance applied to the outer and inner surfaces of the container; characterized in that the ring is substantially planar, the container is substantially elliptical in plan, and the ring and container are such that the ring can be positioned in the container with its plane containing either the major or the minor axis of the elliptical container.

2. Contraceptive vaginal barrier according to claim 1 characterized in that the ring comprises an outer sleeve of natural rubber or equivalent synthetic material into which means are incorporated for allowing the peripheral dimensions of the outer sleeve to be modified as well as giving the outer sleeve a desired shape.

3. Contraceptive vaginal barrier according to claim 1 characterized in that the ring is an annular body, having circular or oval cross section, made of an elastic material such as rubber, synthetic material or steel.

4. Contraceptive vaginal barrier according to claim 1 characterized in that the aperture has a mouth, the edge of which comprises a tubular housing containing a yarn, the ends of which extend out of the ends of the tubular housing in order to be pulled to close the aperture and to be knotted permanently.

5. Contraceptive vaginal barrier according to claim 1 characterized in that the container comprises a second aperture closed by a means which may be removed easily and rapidly for drawing out the ring contained therein.

6. Contraceptive vaginal barrier according to claim 1 characterized in that the container comprises a second aperture closed by a yarn which may be removed easily and rapidly for drawing out the ring contained therein.

7. Contraceptive vaginal barrier according to claim 1 characterized in that the container is made of woven material, flexible plastic material, natural or synthetic rubber, or natural or synthetic sponge.

## Patentansprüche

1. Empfängnisverhütende Scheidensperre, bestehend aus einem kreisförmigen oder ovalen Ring (1); einem abnehmbaren Behälter (2) in Form eines Beutels, der aus flexiblem Material hergestellt ist und eine Öffnung (3), durch welche der Ring eingeführt werden kann, sowie Mittel (4, 5) für das Verschließen der Öffnung aufweist, wobei der Ring und der behälter solche sind, daß der Ring den behälter spannt, wenn er darin eingeführt ist, um so ein im wesentlichen becherförmiges Element, das eine Peripherie aufweist, die der Peripherie des Ringes entspricht, und mit den zwei Behälterwandungen eine auf die andere übereinandergelegt zu bilden; und aus einer spermiziden und/oder medizinischen Substanz, die jeweils auf die äußere und innere Ober fläche des behälters aufgebracht ist; dadurch gekennzeichnet, daß der Ring im wesentlichen planar ist, daß der Behälter im Grundriß im wesentlichen elliptisch ist und daß der Ring und Behälter solche sind, daß der Ring im Behälter mit seiner Ebene, die entweder die größere oder die kleinere Achse des elliptischen Behälters enthält, angeordnet werden kann.

2. Empfängnisverhütende Scheidensperre nach Anspruche 1, dadurch gekennzeichnet, daß der Ring eine äußere Manschette aus natürlichem Gummi oder äquivalentem synthetischen Material aufweist, in welche Mittel eingebaut sind, um zu erlauben, daß sowohl die peripheren Dimensionen der äußeren Manschette modifiziert werden können als auch, um der äußeren Manschette eine gewünschte Form zu verleihen.

3. Empfängnisverhütende Scheidensperre nach Anspruche 1, dadurch gekennzeichnet, daß der Ring ein ringförmiger Körper ist, der einen kreisförmigen oder ovalen Querschnitt aufweist, der aus elastischem Material wie Gummi oder synthetischem Material oder Stahl hergestellt ist.

4. Empfängnisverhütende Scheidensperre nach Anspruche 1, dadurch gekennzeichnet, daß die Öffnung ein Mundstück aufweist, dessen Rand ein rohrförmiges Gehäuse aufweist, das ein Garn enthält, dessen Enden aus den Enden des rohrförmigen Gehäuses herausragen, um zum Verschließen der Öffnung zusammengezogen und um dauerhaft geknotet zu werden.

5. Empfängnisverhütende Scheidensperre nach Anspruche 1, dadurch gekennzeichnet, daß der Behälter eine zweite Öffnung aufweist, die mit einem Mittel verschlossen ist, welches leicht und schnell für das Herausziehen des darin eingeschlossenen Ringes entfernt werden kann.

6. Empfängnisverhütende Scheidensperre nach Anspruche 1, dadurch gekennzeichnet, daß der behälter eine zweite Öffnung aufweist, die mit einem Garn verschlossen ist, welches leicht und schnell für das Herausziehen des darin eingeschlossenen Ringes entfernt werden kann.

7. Empfängnisverhütende Scheidensperre nach Anspruche 1, dadurch gekennzeichnet, daß der Behälter aus gewebtem Material, flexiblem Plastikmaterial, natürlichem oder synthetischem Gummi oder natürlichem oder synthetischem Schwamm hergestellt ist.

## Revendications

1. Barrage vaginal contraceptif comprenant un anneau circulaire ou ovale (1); un récipient jetable (2) sous la forme d'un sac formé d'une matière flexible, présentant une ouverture (3) à travers laquelle on peut introduire l'anneau et des moyens (4, 5) pour fermer l'ouverture, l'anneau et le récipient étant tels que l'anneau dilate le récipient lorsqu'on l'y introduit de manière à former un élément pratiquement en forme de cuvette ayant une périphérie correspondant pratiquement à la périphérie de l'anneau et dans lequel les deux parois du récipient sont supeposées l'une à l'autre; et une substance spermicide et/ou médicinale appliquée aux surfaces extérieure et intérieure du récipient; caractérisé par le fait que l'anneau est pratiquement plan, que le récipient est pratiquement elliptique dans le plant et que l'anneau et le récipient sont tels que l'anneau peut être positionné dans le récipient, son plan contenant soit le petit axe du récipient elliptique.

2. Barrage vaginal contraceptif selon la revendication 1, caractérisé par le fait que l'anneau comprend un manchon extérieur en caoutchoic naturel ou matière synthétique équivalente auquel sont incorporés des moyens pour permettre de modifier les dimensions périphériques du manchon extérieur, ainsi que pour donner au manchon extérieur une forme désirée.

3. Barrage vaginal contraceptif selon la revendication 1, caractérisé par le fait que l'anneau est un corps annulaire ayant une section circulaire ou ovale, formé d'une matière élastique telle que le caoutchouc, une matière synthétique ou l'acier.

4. Barrage vaginal contraceptif selon la revendication 1, caractérisé par le fait que l'ouverture présente une embouchure dont le bord constitue une enveloppe tubulaire contenant un fil dont les extrémités s'étendent hors des extrémités de l'enveloppe tubulaire afin d'être tirées pour fermer l'ouverture et d'être nouées de façon permanente.

5. Barrage vaginal contraceptif selon la revendication 1, caractérisé par le fait que le récipient comprend une deuxième ouverture fermée par un moyen qui peute être enlevé facilement et rapidement pour retirer l'anneau qui y est contenu.

6. Barrage vaginal contraceptif selon la revendication 1, caractérisé par le fait que le récipient comprend une deuxième ouverture fermée par un fil qui peut être enlevé facilement et rapidement pour retirer l'anneau qui y est contenu.

7. Barrage vaginal contraceptif selon la revendication 1, caractérisé par le fait que le récipient est formé de matière tissée, de matière plastique flexible, de caoutchouc naturel ou synthétique ou d'éponge naturelle ou synthétique.

0 081 131

FIG. 1

FIG. 2

FIG. 3

FIG. 4

1